# EUROPEAN PATENT APPLICATION

(11) **EP 3 624 128 A1**
(43) Date of publication of application: **18.03.2020**
(21) Application number: 18194906.6
(22) Date of filing: 17.09.2018
(51) Int. Cl.: G16H 20/00, G06F 17/27

(54) **AN APPARATUS AND METHOD FOR DETECTING AN INCIDENTAL FINDING**

(71) Applicant: Koninklijke Philips N.V., 5656 AG Eindhoven (NL)
(72) Inventor: TAHMASEBI MARAGHOOSH, Amir Mohammad, 5656 AE Eindhoven (NL); MANKOVICH, Gabriel Ryan, 5656 AE Eindhoven (NL)
(74) Representative: de Haan, Poul Erik

(57) **Abstract**

There is provided a computer-implemented method for detecting an incidental finding in a radiology report associated with an imaging examination of a subject. The method comprises acquiring a first finding from the radiology report, the first finding being indicative of at least one of: an imaging observation, a disorder, and an abnormality identified from the imaging examination, acquiring information associated with the radiology report, wherein the acquired information associated with the radiology report comprises at least one of: a reason for performing the imaging examination, a modality of the imaging examination, and a body part of the subject examined in the imaging examination, and determining whether the first finding is an incidental finding based on the acquired information associated with the radiology report, wherein the incidental finding is an unanticipated finding not related to a diagnostic inquiry associated with the imaging examination.

## Description

### FIELD OF THE INVENTION

The present disclosure relates to an apparatus and method for detecting an incidental finding. In particular, the present disclosure relates to an apparatus and method for detecting an incidental finding in a radiology report associated with an imaging examination of a subject.

### BACKGROUND OF THE INVENTION

Radiologists diagnose diseases and provide statuses for diseases after reviewing reports resulting from imaging examinations. Specifically, in a radiology workflow, a radiologist reviews a set of images from a medical imaging examination and dictate his/her observations as well as provide his/her impression and diagnosis for potential next steps. Typically, radiology reports include results of a reading of a medical imaging examination and also information regarding suggested follow-up actions recommended by the radiologist. The term "radiologist" is used throughout this description to refer to an individual who is reviewing a patient's medical records, but it will be apparent to those skilled in the art that the individual may alternatively be any other appropriate user, such as a doctor, a nurse, or other medical professional.

In some instances, radiologists may come across a finding that is not directly related to the original reason the imaging examination was requested. This type of finding is referred to as an incidental finding. Detection and distinction of incidental findings in radiology reports can be a challenging task. Sometimes patients with incidental findings are either missed from the appropriate care management programs such as lung cancer screening program, or mistakenly added to inappropriate clinical workflows, which may result in complications for the patient as well as unnecessary workload and costs for the hospital and the care team.

It is therefore important to accurately detect incidental findings and to determine whether follow-up actions are required for incidental findings based on the clinical significance of the finding, since the lack of follow-up actions may potentially result in health deterioration of the patient. Exemplary follow-up actions may include further imaging studies to improve understanding of the clinical problem, or to detect clinical changes of the patient over time. Attentive management of incidental findings following identification of the findings may lead to early diagnosis and treatment of diseases, and the failure to carry out follow-up actions may negatively impact patient clinical outcomes. There has been an exponential increase in imaging volumes and as a result, increase in the number of reports generated for these imaging examinations. Hence, there has been a significant increase in the number of incidental findings being reported. However, it has been shown that for around 30% of cases in which incidental findings are observed, no follow-up action is provided.

### SUMMARY OF THE INVENTION

As noted above, there has been significant interest in the mechanization of the capture of incidental findings and their associated follow-up actions. It would therefore be advantageous to provide an improved method and apparatus for detecting an incidental finding in a radiology report and also for providing an appropriate workflow for a patient when an incidental finding is detected, so as to ensure that appropriate care is provided to the patient in a timely manner.

To better address one or more of the concerns mentioned earlier, in a first aspect, there is provided a computer-implemented method for detecting an incidental finding in a radiology report associated with an imaging examination of a subject. The method comprises acquiring a first finding from the radiology report, the first finding being indicative of at least one of: an imaging observation, a disorder, and an abnormality identified from the imaging examination; acquiring information associated with the radiology report, wherein the acquired information associated with the radiology report comprises at least one of: a reason for performing the imaging examination, a modality of the imaging examination, and a body part of the subject examined in the imaging examination; and determining whether the first finding is an incidental finding based on the acquired information associated with the radiology report, wherein the incidental finding is an unanticipated finding not related to a diagnostic inquiry associated with the imaging examination.

In some embodiments, acquiring the first finding from the radiology report may comprise performing keyword-based matching using a predetermined list of keywords.

In some embodiments, each of the predetermined list of keywords may be extracted from at least one of domain knowledge and available standard ontologies.

In some embodiments, acquiring the first finding from the radiology report may be based on a trained model. In these embodiments, the model may be trained for identifying one or more findings from radiology reports, using at least one of machine learning processes and deep learning processes, and the model may be trained based on a plurality of human annotated radiology reports, each of the plurality of human annotated radiology reports comprising one or more findings. Furthermore, in these embodiments, at least one of the machine learning processes and deep learning processes may be based on one of: logistic regression, support vector machine, random forest, convolutional neural networks, and recurrent neural networks.

In some embodiments, the radiology report may comprise a plurality of sections, each of the plurality of sections being associated with a finding or a type of information associated with the radiology report. In these embodiments, acquiring the first finding or the information associated with the radiology report may comprise: identifying a relevant section in the radiology report among the plurality of sections; and using natural language processes to extract words and/or phrases from the identified section, wherein the words and/or phrases are relevant to the first finding or the information associated with the radiology report.

In some embodiments, the method may further comprise acquiring a clinical history of the subject. In these embodiments, determining whether the first finding is an incidental finding is further based on the acquired clinical history of the subject.

In some embodiments, the method may further comprise acquiring a second finding from a previously radiology report associated with the subject, wherein the second finding of the same pathological type as that associated with the first finding. In these embodiments, determining whether the first finding is an incidental finding may be further based on a comparison between the first finding and the second finding.

In some embodiments, the method further comprises acquiring, for each of the first finding and the second finding, one or more features. In these embodiments, each of the one or more features may be indicative of a parameter of an observed element associated with the respective finding, and the comparison between the first finding and the second finding may comprise comparing the one or more features associated with the first finding with the one or more features associated with the second finding. Moreover, in these embodiments, a parameter of an observed element may be one of: an anatomical location of the observed element, a size of the observed element, a shape of the observed element, margin information of the observed element, a degree of opacity of the observed element, and a texture of the observed element.

In some embodiments, if it is determined that the first finding is an incidental finding, the method may further comprise: acquiring a clinical guideline, wherein the clinical guideline corresponds to the acquired information associated with the radiology report and comprises a decision support tool; acquiring one or more features associated with the first finding, wherein each of the one or more features is indicative of a parameter of an observed element associated with the first finding; and determining whether a follow-up action or a follow-up recommendation is required for the first finding, based on the acquired one or more features associated with the first finding and the decision support tool of the acquired clinical guideline.

In some embodiments, the method may further comprise acquiring information associated with the subject. In these embodiments, determining whether a follow-up action or a follow-up recommendation is required for the first finding may be further based on the acquired information associated with the subject.

In some embodiments, the modality of the imaging examination may be one of: a computed tomography scan, a magnetic resonance imaging scan, an ultrasound scan, and an X-ray scan.

In a second aspect, there is provided a computer program product comprising a computer readable medium, the computer readable medium having computer readable code embodied therein, the computer readable code being configured such that, on execution by suitable computer or processor, the computer or processor is caused to perform the method as described above.

In a third aspect, there is provided an apparatus detecting an incidental finding in a radiology report associated with an imaging examination of a subject. The apparatus comprises a processor configured to: acquire a first finding from the radiology report, the first finding being indicative of at least one of: an imaging observation, a disorder, and an abnormality identified from the imaging examination; acquire information associated with the radiology report, wherein the acquired information associated with the radiology report comprises at least one of: a reason for performing the imaging examination, a modality of the imaging examination, and a body part of the subject examined in the imaging examination; and determine whether the first finding is an incidental finding based on the acquired information associated with the radiology report, wherein the incidental finding is an unanticipated finding not related to a diagnostic inquiry associated with the imaging examination.

According to the aspects and embodiments described above, the limitations of existing techniques are addressed. In particular, the above-described aspects and embodiments enable incidental findings to be detected accurately and allow detected incidental findings to be managed effectively by determining whether follow-up actions and/or recommendations are required based on corresponding clinical guidelines.

There is thus provided an improved method and apparatus for detecting an incidental finding in a radiology report associated with an imaging examination of a subject. These and other aspects of the disclosure will be apparent from and elucidated with reference to the embodiment(s) described hereinafter.

### BRIEF DESCRIPTION OF THE DRAWINGS

For a better understanding of the embodiments, and to show more clearly how they may be carried into effect, reference will now be made, by way of example only, to the accompanying drawings, in which:
Fig. 1 is a block diagram of an apparatus for detecting an incidental finding in a radiology report associated with an imaging examination of a subject, according to an embodiment;
Fig. 2 illustrates a method for detecting an incidental finding in a radiology report associated with an imaging examination of a subject, according to an embodiment; and
Fig. 3 is a workflow diagram illustrating a method for detecting an incidental finding in a radiology report associated with an imaging examination of a subject, according to an embodiment.

### DETAILED DESCRIPTION OF EMBODIMENTS

As noted above, there is provided an improved apparatus and a method of operating the same which addresses the existing problems.

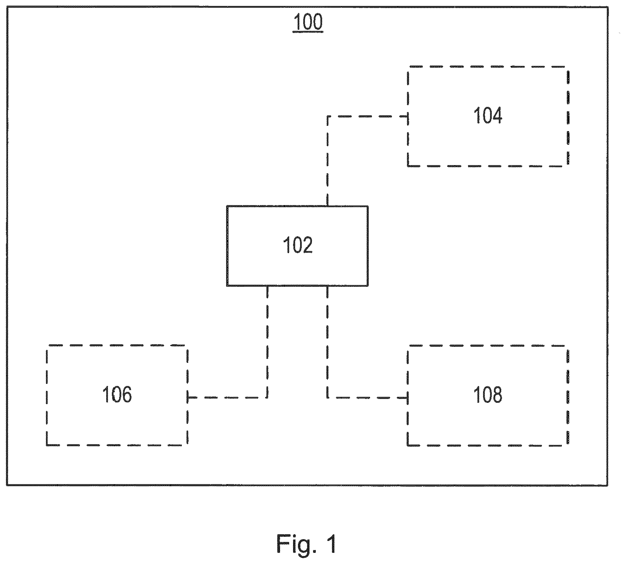

**Fig. 1** shows a block diagram of an apparatus 100 according to an embodiment, which can be used for detecting an incidental finding in a radiology report associated with an imaging examination of a subject. A radiology report comprises written description prepared by a radiologist who interprets the imaging study of the imaging examination and/or a physician who requested the imaging examination, and a finding in the radiology report is indicative of at least one of: an imaging observation identified from the imaging examination, a disorder identified from the imaging examination, and an abnormality identified from the imaging examination.

The imaging examination may be an examination performed on magnetic resonance imaging (MRI), computed tomography (CT), positron emission chromatography (PET), ultrasound, etc. These types of imaging examinations are referred to as "modalities" in the description herein. It will be appreciated the method and the apparatus described in the present disclosure may be used to detect an incidental finding for radiology reports associated with any type of imaging examination.

As illustrated in Fig. 1, the apparatus comprises a processor 102 that controls the operation of the apparatus 100 and that can implement the method described herein. The processor 102 can comprise one or more processors, processing units, multi-core processor or modules that are configured or programmed to control the apparatus 100 in the manner described herein. In particular implementations, the processor 102 can comprise a plurality of software and/or hardware modules that are each configured to perform, or are for performing, individual or multiple steps of the method described herein.

Briefly, the processor 102 is configured to acquire a first finding from the radiology report. As mentioned above, the first finding is indicative of at least one of: an imaging observation identified from the imaging examination, a disorder identified from the imaging examination, and an abnormality identified from the imaging examination. The processor 102 is also configured to acquire information associated with the radiology report. The acquired information associated with the radiology report comprises at least one of: a reason for performing the imaging examination, a modality of the imaging examination, and a body part of the subject examined in the imaging examination. The processor 102 is further configured to determine whether the first finding is an incidental finding based on the acquired information associated with the radiology report. An incidental finding is an unanticipated finding not related to a diagnostic inquiry associated with the imaging examination, for example an observed pulmonary nodule in a radiology report where the diagnostic inquiry of the imaging examination is not concerned with the lungs of the subject. Other examples of findings may include a breast lesion, or a bone lesion, etc.

In some embodiments, the apparatus 100 may further comprise at least one user interface 104. Alternative or in addition, at least one user interface 104 may be external to (i.e. separate to or remote from) the apparatus 100. For example, at least one user interface 104 may be part of another device. A user interface 104 may be for use in providing a user of the apparatus 100 with information resulting from the method described herein. Alternatively or in addition, a user interface 104 may be configured to receive a user input. For example, a user interface 104 may allow a user of the apparatus 100 to manually enter instructions, data, or information. In these embodiments, the processor 102 may be configured to acquire the user input from one or more user interfaces 104.

A user interface 104 may be any user interface that enables the rendering (or output or display) of information to a user of the apparatus 100. Alternatively or in addition, a user interface 104 may be any user interface that enables a user of the apparatus 100 to provide a user input, interact with and/or control the apparatus 100. For example, the user interface 104 may comprise one or more switches, one or more buttons, a keypad, a keyboard, a touch screen or an application (for example, on a tablet or smartphone), a display screen, a graphical user interface (GUI) or other visual rendering component, one or more speakers, one or more microphones or any other audio component, one or more lights, a component for providing tactile feedback (e.g. a vibration function), or any other user interface, or combination of user interfaces.

In some embodiments, the apparatus 100 may comprise a memory 106. Alternatively or in addition, one or more memories 106 may be external to (i.e. separate to or remote from) the apparatus 100. For example, one or more memories 106 may be part of another device. A memory 106 can be configured to store program code that can be executed by the processor 102 to perform the method described herein. A memory can be used to store information, data, signals and measurements acquired or made by the processor 102 of the apparatus 100. For example, a memory 106 may be used to store (for example, in a local file) one or more radiology reports associated with the subject. The processor 102 may be configured to control a memory 106 to store one or more radiology reports associated with the subject.

In some embodiments, the apparatus 100 may comprise a communications interface (or circuitry) 108 for enabling the apparatus 100 to communicate with any interfaces, memories and/or devices that are internal or external to the apparatus 100. The communications interface 108 may communicate with any interfaces, memories and/or devices wirelessly or via a wired connection. For example, the communications interface 108 may communicate with one or more user interfaces 104 wirelessly or via a wired connection. Similarly, the communications interface 108 may communicate with the one or more memories 106 wirelessly or via a wired connection.

Although the operation of the apparatus 100 is described above in the context of a single finding in a particular radiology report, it will be appreciated that the apparatus 100 is capable of performing detection of incidental finding(s) for more than one findings contained in one or more radiology reports. Moreover, it will be appreciated that Fig. 1 only shows the components required to illustrate an aspect of the apparatus 100 and, in a practical implementation, the apparatus 100 may comprise alternative or additional components to those shown.

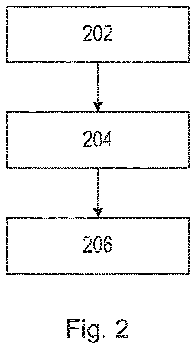

**Fig. 2** illustrates a computer-implemented method for detecting an incidental finding in a radiology report associated with an imaging examination of a subject, according to an embodiment. The illustrated method can generally be performed by or under the control of processor 102 of the apparatus 100.

With reference to Fig. 2, at block 202, a first finding from the radiology report is acquired. The processor 102 of the apparatus 100 may be configured to acquire the first finding from the radiology report which is stored in the memory 106 of the apparatus 100. As mentioned with reference to Fig. 1 above, the first finding is indicative of at least one of: an imaging observation (e.g. a lymph node) identified from the imaging examination, a disorder (e.g. cancer) identified from the imaging examination, and an abnormality (e.g. pulmonary nodules) identified from the imaging examination. In some embodiments, the imaging observation, the disorder, or the abnormality may be included in the radiology report based on dictation by a radiologist.

In some embodiments, acquiring the first finding from the radiology report at block 202 may comprise performing keyword-based matching using a predetermined list of keywords. Each of the predetermined list of keywords may be extracted from at least one of domain knowledge and available standard ontologies. For example, each of the predetermined list of keywords may be extracted from Systematized Nomenclature of Medicine - Clinical Terms (SNOMED CT), which is a computer-processable collection of medical terms providing codes, terms, synonyms and definitions used in clinical documentation and reporting (e.g. radiology reports). As another example, each of the predetermined list of keywords may be extracted from RadLex, which is a database comprising standard terms for radiology reporting, terms used by structured radiology reporting templates, standard codes and names of imaging examinations, etc. Alternatively or in addition, other types of databases may be used for extracting the predetermined list of keywords.

Moreover, in some embodiments, acquiring the first finding from the radiology report at block 202 may be based on a trained model. The model may be trained for identifying one or more findings from radiology reports, using at least one of machine learning processes and deep learning processes. In these embodiments, the model may be trained based on training data such as a plurality of radiology reports each comprising one or more findings, wherein the findings may be highlighted by human annotation). Furthermore, in these embodiments, at least one of the machine learning processes and deep learning processes may be based on one of: logistic regression, support vector machine, random forest, convolutional neural networks, and recurrent neutral networks.

In some embodiments, the radiology report associated with the subject may comprise a plurality of sections. Each of the plurality of sections may be associated with a finding or a type of information associated with the radiology report. In these embodiments, acquiring the first finding from the radiology report at block 202 may comprise identifying a relevant section in the radiology report associated with the subject among the plurality of sections in the radiology report, and using natural language processes to extract, from the identified section, words and/or phrases that are relevant to the first finding.

For example, the radiology report associated with the subject may comprise the plurality of fixed sections including: "Type of exam", "Clinical history", "Comparison", "Technique", "Findings", and "Impression". In this example, the section "Findings" may include one or more sentences related to the observations regarding the body part examined in the imaging examination, such as the identification of a pulmonary nodule. Accordingly, in this example, the first finding may be acquired by identifying the "Finding" section in the radiology report and using natural language processes to extract a phrase or a sentence which mentions the identified pulmonary nodule. Furthermore, the extraction of words and/or phrases that are relevant to the first finding may be based on identifying co-occurrence of specific words or phrases, such as the co-occurrence of words "nodule", "pulmonary", "new", in the same sentence in a relevant section of the radiology report, or in the same paragraph in a relevant section of the radiology report, or in the same relevant section of the radiology report, or in the same radiology report.

In some embodiments, rule-based approaches or grammar-based approaches may be used for identifying the relevant section in the radiology report. For example, a rule-based approach or a grammar-based approach may include the use of logic language (e.g. "and" or "or" conditions) that dictates how a relevant section is to be identified, on the basis of a list of detected keywords (e.g. "new", "nodule") or acquired features. It will be explained in more detail below how feature(s) can be detected/acquired. In some embodiments, a rule-based approach or a grammar-based approach may include negation detection. For example, occurrence of specific words or phrases such as "stable" or "seen" in a sentence may indicate that the finding associated with the sentence is unlikely to qualify as an incidental finding, and therefore in this case the processor 102 may be configured, based on this negation detection rule, to exclude this particular sentence from being extracted as (at least part of) the first finding.

Returning back to Fig. 2, at block 204, information associated with the radiology report is acquired. This information may be acquired by the processor 102 of the apparatus 100. The information associated with the radiology report comprises at least one of: a reason for performing the imaging examination, a modality of the imaging examination, and a body part of the subject examined in the imaging examination. The modality of the imaging examination may be one of: a computer tomography (CT) scan, a magnetic resonance imaging (MRI) scan, an ultrasound scan, and an X-ray scan.

In some embodiments, the information associated with the radiology report may be presented in a standardized format. For example, in some embodiments the reason for performing the imaging examination may only be input (e.g. by the radiologist) as one of the plurality of predetermined categories such as "follow-up examination", "accident", etc.; the modality of the imaging examination may only be input as one of the plurality of predetermined categories such as "computer tomography (CT)", "magnetic resonance imaging (MRI)", "ultrasound", etc.; and the body part of the subject examined in the imaging examination may only be input as one of the plurality of categories such as "abdomen", "pelvis", etc. Hence, in this case, the standardized format allows the information associated with the radiology report to be extracted or acquired by the processor 102 more accurately and efficiently.

In some embodiments, acquiring the information associated with the radiology report at block 204 may be based on a trained model for identifying at least one of: a reason for performing the imaging examination, a modality of the imaging examination, and a body part of the subject examined in the imaging examination. The model may be trained using at least one of machine learning processes and deep learning processes. For example, the model may be trained based on training data such as a plurality of human annotated radiology reports, each radiology report comprising at least one of: a reason for performing the imaging examination, a modality of the imaging examination, and a body part of the subject examined in the imaging examination. In some cases, the relevant information may be highlighted (e.g. using annotation) in the radiology reports so as to increase the effectiveness of the training of the model. Furthermore, in these embodiments, at least one of the machine learning processes and deep learning processes may be based on one of: conditional random field, logistic regression, support vector machine, random forest, convolutional neural networks, and recurrent neutral networks.

As mentioned with reference to block 202 above, in some embodiments the radiology report associated with the subject may comprise a plurality of sections, and that in these embodiments each of the plurality of sections may be associated with a finding or a type of information associated with the radiology report. In these embodiments, acquiring the information associated with the radiology report at block 204 may comprise identifying a relevant section in the radiology report associated with the subject, among the plurality of sections in the radiology report, and using natural language processes to extract, from the identified section, words and/or phrases that are relevant to the information associated with the radiology report. In these embodiments, rule-based approaches or grammar-based approaches may be used for identifying the relevant section.

In more detail, as mentioned above, in some embodiments the radiology report associated with the subject may comprise the plurality of fixed sections, such as the section "Type of exam" which may include information such as the date of the imaging examination, the time of the imaging examination, or the type of imaging study (i.e. modality) that was performed. As an example, the "Type of exam" section may include the description: "Computer tomography (CT) of the abdomen and pelvis with intravenous and oral contrast performed on 10 January 2018". In this example, the modality of the imaging examination may be acquired by identifying the "Type of exam" section in the radiology report, and subsequently using natural language processes to extract the relevant phrase ("Computer tomography") in the identified section. Also, in this example, the body part examined in the imaging examination may be acquired by identifying the same section in the radiology report and using natural language processes to extract the relevant word ("abdomen" and/or "pelvis") in the identified section. It will be appreciated that in some cases more than one body parts examined in the imaging examination may be acquired as the information associated with the radiology report at block 204.

In some embodiments, the information associated with the radiology report may be contained in a header part of the radiology report associated with the subject. For example, if the radiology report is in the Digital Imaging and Communications in Medicine (DICOM) standard, the modality of the imaging examination may be contained in the DICOM header of the radiology report. Therefore, in these embodiments, acquiring information associated with the radiology report at block 204 may comprise extracting the modality of the imaging examination from the header part of the radiology report.

Returning back to Fig. 2, at block 206, it is determined, based on the information associated with the radiology report acquired at block 204, whether the first finding is an incidental finding. This determination may be performed by the processor 102 of the apparatus 100. Determining whether the first finding is an incidental finding may be based on one or more conditions to be satisfied, as will be explained in more detail below.

As an example, if the information associated with the radiology report acquired at block 204 comprises "computed tomography" as the modality of the imaging examination and "abdomen and pelvis" as the body parts of the subject examined in the imaging examination, it is expected that the finding(s) in the radiology report are associated with observations of the abdomen and pelvis. In this case, if the first finding is associated with the chest area, it is likely that the first finding is an incidental finding. On the other hand, if the first finding is associated with the abdomen area or the pelvis area, it is unlikely that the first finding is an incidental finding. Therefore, in some embodiments, the processor 102 may be configured to determine whether the first finding is an incidental finding by comparing an anatomical location associated with the first finding and the body part(s) examined in the imaging examination.

In some cases, one of the further conditions that needs to be satisfied for the first finding to be determined as an incidental finding is that the first finding is a new finding for the subject that has not been observed previously. Accordingly, in some embodiments, the method for detecting an incidental finding in a radiology report associated with an imaging examination of a subject may further comprise acquiring a clinical history of the subject. The clinical history of the subject may be indicative of information associated with the subject such as age, sex, existing diseases and symptoms, a known or a suspected diagnosis, etc. In these embodiments, determining whether the first finding is an incidental finding at block 206 may be further based on the acquired clinical history of the subject. In this case, the processor 102 may determine whether the first finding is not an incidental finding based on whether the clinical history of the subject comprises information (e.g. existing symptoms) that indicates that a same finding or a similar finding has been observed previously for the subject. Alternatively or in addition, the processor 102 may determine whether the first finding is an incidental finding based on whether the clinical history of the subject comprises information (e.g. a suspected diagnosis) that indicates a similar finding has been observed previously for the subject but the previous finding was not deemed as actionable (i.e. requiring a follow-up action or a follow-up recommendation), such as a micro nodule that was less than 2mm.

Moreover, in these embodiments, the clinical history of the subject may be acquired in a similar manner as the way the information associated with the radiology report is acquired at block 204. In some embodiments, the clinical history of the subject may be acquired simultaneously with the information associated with the radiology report at block 204.

For example, in some embodiments, the clinical history of the subject may be associated with one of the plurality of sections in the radiology report associated with the subject. Therefore, in these embodiments, acquiring the clinical history of the subject may comprise identifying a relevant section among the plurality of sections in the radiology report and using natural language processes to extract words and/or phrases that relevant to the clinical history of the subject from the identified section. For example, the radiology report associated with the subject may comprise the plurality of fixed sections, such as the section "Clinical history" which may include information associated with the subject such as age, sex, existing diseases and symptoms, a known or a suspected diagnosis, etc. In this example, the "Clinical history" section may include the description: "60 year-old female with history of breast cancer and new onset of abdominal pain". Accordingly, the clinical history of the subject may be acquired by identifying the "Clinical history" section in the radiology report and using natural language processes to extract the relevant phrase ("history of breast cancer") in the identified section. In addition, in this example the processor 102 may be configured to determine whether the first finding (e.g. a breast lesion) is an incidental finding based on the fact that the subject has a history of breast cancer.

In some embodiments, the method for detecting an incidental finding in a radiology report associated with an imaging examination of a subject may further comprise acquiring a second finding from a previous radiology report associated with the subject, the second finding being of the same pathological type as that associated with the first finding. For example, two findings may be considered as being of the same pathological type when both of them are associated with an identified mass in the same anatomical location. In these embodiments, determining whether the first finding is an incidental finding at block 206 may be further based on a comparison between the first finding and the second finding.

As an example of acquiring the second finding which is of the same pathological type as that associated with the first finding, if the first finding is associated with an observed nodule in the left upper lobe of the lung, the processor 102 may be configured to perform a search query in the previous radiology report(s) of the subject to acquire a second finding that is also associated with a similar or the same anatomical location, i.e. an observed nodule located in the left upper lobe of the lung. In the case multiple findings in previous radiology report(s) are found, the processor 102 may be configured to perform an additional search query using location coordinates.

In addition, in these embodiments, the method may further comprise acquiring, for each of the first finding and the second finding, one or more features. Each of the one or more features may indicative of a parameter of an observed element associated with the respective finding. Specifically, in some embodiments, a parameter of an observed element may be one of: an anatomical location of the observed element, a size of the observed element, a shape of the observed element, margin information of the observed element, a degree of opacity of the observed element, and a texture of the observed element. In these embodiments, the comparison between the first finding and the second finding may comprise comparing the one or more features associated with the first finding with the one or more features associated with the second finding. Specifically, corresponding features of the first finding and the second finding may be compared.

In some embodiments, acquiring the one or more features for respective findings may be based on a trained model. The model may be trained using at least one of machine learning processes and deep learning processes. For example, the model may be trained based on training data such as a plurality of findings each comprising one or more features that are highlighted. Furthermore, in these embodiments, at least one of the machine learning processes and deep learning processes may be based on one of: conditional random field, logistic regression, support vector machine, random forest, convolutional neural networks, and recurrent neutral networks.

In some embodiments, acquiring the one or more features may be based on a predetermined classification framework. For example, features of findings that are associated with a pulmonary nodule may be classified as one of: a solid nodule, a partially solid nodule, and a non-solid nodule. These three classes may be represented using numbers, i.e. "0" representing a solid nodule, "1" representing a partially solid nodule, and "2" representing a non-solid nodule. In this example, therefore, a feature of a respective finding may be acquired by extracting the class (and/or a representative descriptor of the class) associated with the finding, e.g. extracting the phrase "partially solid" as the feature or interpreting the number "2" as "partially solid", if the respective finding is associated with a pulmonary nodule. In some embodiments, acquiring the one or more features may be based on at least one of textual information in the radiology report (e.g. description of a pulmonary nodule identified based on image data) and clinical guidelines (e.g. ACR clinical practice guidelines.

In some embodiments, determining whether the first finding is an incidental finding at block 206 may be based on a model trained for detecting an incidental finding, using at least one of machine learning processes and deep learning processes. In these embodiments, the model may be trained based on training data such as a plurality of previous incidental findings and non-incidental findings, one or more features of the findings, and information associated with the respective previous radiology reports. Furthermore, in these embodiments, at least one of the machine learning processes and deep learning processes may be based on one of: logistic regression, support vector machine, random forest, convolutional neural networks, and recurrent neutral networks.

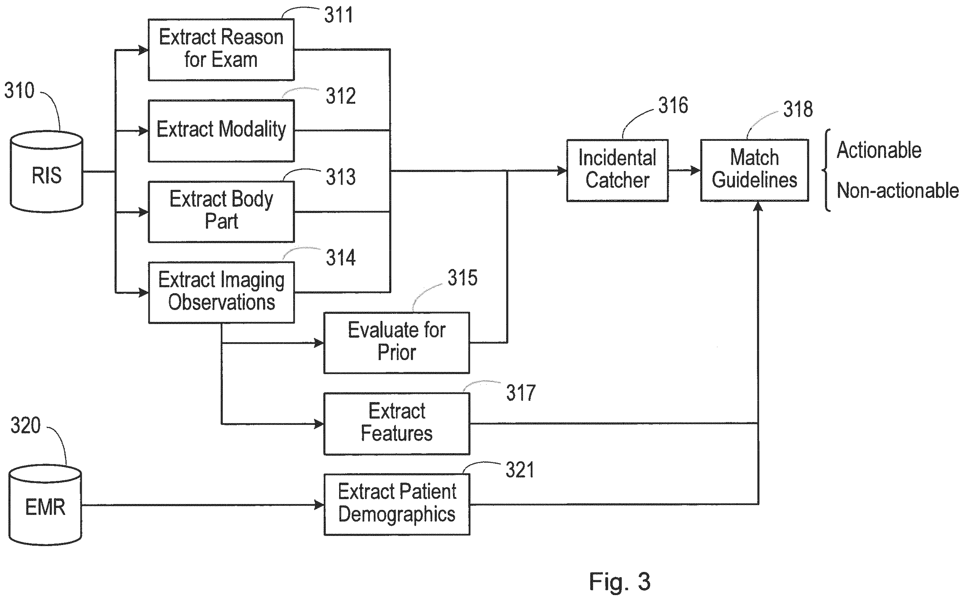

Although not shown in Fig. 2, in some embodiments, the method may further comprise acquiring a clinical guideline that corresponds to the information associated with the radiology report. This may be referred to as a "matching clinical guideline", for example with reference to Fig. 3.

The clinical guideline may be acquired or extracted from the clinical practice guidelines developed and/or endorsed by the American College of Rheumatology (ACR), such as specific guidelines rheumatoid arthritis, osteoarthritis, gout, extrarenal lupus, etc. These clinical practice guidelines enable effective use of health care resources by providing guidance for particular patterns of practice in the medical field. In some cases, the acquired clinical guideline may comprise a decision support tool, such as a decision tree. The decision tree may comprise a plurality of rules that may aid a medical personnel (e.g. a radiologist) in determining whether a follow-up action or a follow-up recommendation is required. Examples of follow-up actions or follow-up recommendations include performing or recommending further imaging studies of the subject, and monitoring clinical changes of the subject over a predetermined period of time.

As mentioned above, in some embodiments the method may further comprise acquiring one or more features associated with the first finding, each of the one or more features being indicative of a parameter of an observed element associated with the first finding. In these embodiments, the method may also further comprise determining whether a follow-up action or a follow-up recommendation is required for the first finding, based on the acquired one or more features associated with the first finding and the acquired clinical guideline. In addition, although not shown in Fig. 2, in some embodiments the method for detecting an incidental finding in a radiology report associated with an imaging examination of a subject may further comprise acquiring information associated with the subject, such as the age of the subject, the sex of the subject, the family history of cancer of the subject, etc. In these embodiments, determining whether a follow-up action or a follow-up recommendation may be further based on the acquired information associated with the subject. The case in which a follow-up action or a follow-up recommendation is required may be referred to as an actionable incidental finding, and the case in which a follow-up action or a follow-up recommendation is not required may be referred to as a non-actionable incidental finding.

**Fig. 3** is a workflow diagram illustrating a method for detecting an incidental finding in a radiology report associated with an imaging examination of a subject, according to an embodiment. The illustrated method can generally be performed by or under the control of processor 102 of the apparatus 100.

As shown in Fig. 3, in the workflow diagram there is provided a radiology information system (RIS) 310 and an electronic health record (EMR) database 320. The radiology information system 310 comprises a plurality of radiology reports associated with one or more subjects, and the electronic health record database 320 comprises information associated with the one or more subjects.

In the embodiment as illustrated in Fig. 3, the workflow comprises, for a radiology report associated with a subject stored in the radiology information system 310, extracting a reason for performing the imaging examination at block 311, extracting a modality of the imaging examination at block 312, extracting a body part examined in the imaging examination at block 313, and extracting imaging observations at block 314. It will be appreciated that in some embodiments the method may not necessarily include extracting the reason for the imaging examination, or extracting the modality of the imaging examination, or extracting the body part examined in the imaging examination, and it is sufficient to only extract one type of information associated with the radiology report. The imaging observations extracted at block 314 are herein referred to as "current finding(s)". As described above with reference to Fig. 2, the extraction of information, e.g. extracting a reason for performing the imaging examination at block 311, may be based on a model trained using at least one of machine learning processes and deep learning processes.

The process of extracting imaging observations at block 314 may be considered to correspond to block 202 of the method as illustrated in Fig. 2. Also, the processes of extracting the reason for performing the imaging examination at block 311, extracting the modality of the imaging examination at block 312, and extracting the body part examined in the imaging examination at block 313 may be considered to collectively correspond to block 204 of the method as illustrated in Fig. 2. Hence, the processes at blocks 311 to 314 may be performed by the processor 102 of the apparatus 100 in the manner as described with reference to Fig. 2 above.

As shown in Fig. 3, the workflow further comprises, at block 315 ("Evaluate for prior"), extracting finding(s) from previous radiology report(s) associated with the subject that are similar to the current finding(s) resulting from the process at block 314. Subsequently, the results from blocks 311 to 315, which include the similar findings from previous radiology report(s) and at least one of: the reason for performing the imaging examination, the modality of the imaging examination, and the body part of the subject examined in the imaging examination, are used at block 316 ("Incidental Catcher") for the determination of whether any of the current finding(s) is an incidental finding. The process at block 316 may be considered to correspond to block 206 of the method illustrated in Fig. 2. Hence, the process at block 316 may be also be performed by the processor 102 of the apparatus 100 in the manner as described with reference to Fig. 2 above.

As shown in Fig. 3, the workflow further comprises extracting, at block 317, feature(s) of the incidental finding if at block 316 it is determined that at least one of the current findings is an incidental finding, and extracting information associated with the subject, such as the age of the subject, the sex of the subject, the family history of cancer of the subject, from the electronic health record database 310 at block 321 ("Extract Patient Demographics"). The incidental finding detected at block 316, the features of the incidental finding extracted at block 317, and the information associated with the subject extracted at block 312 are then used for determining whether the incidental finding is actionable or non-actionable at block 318 ("Match Guidelines"), based on clinical guidelines such as ACR clinical practice guidelines, in the manner as described with reference to Fig. 2 above.

There is thus provided an improved method and apparatus for detecting an incidental finding in a radiology report associated with an imaging examination of a subject, which overcomes the existing problems.

There is also provided a computer program product comprising a computer readable medium, the computer readable medium having computer readable code embodied therein, the computer readable code being configured such that, on execution by a suitable computer or processor, the computer or processor is caused to perform the method or methods described herein. Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. In the present disclosure, the expression "at least one of A, B and C" means "A, B, and/or C", and that it suffices if e.g. only B is present. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer-implemented method for detecting an incidental finding in a radiology report associated with an imaging examination of a subject, the method comprising:
acquiring a first finding from the radiology report, the first finding being indicative of at least one of: an imaging observation, a disorder, and an abnormality identified from the imaging examination;
acquiring information associated with the radiology report, wherein the acquired information associated with the radiology report comprises at least one of: a reason for performing the imaging examination, a modality of the imaging examination, and a body part of the subject examined in the imaging examination; and
determining whether the first finding is an incidental finding based on the acquired information associated with the radiology report, wherein the incidental finding is an unanticipated finding not related to a diagnostic inquiry associated with the imaging examination.

2. A computer-implemented method according to claim 1, wherein acquiring the first finding from the radiology report comprises performing keyword-based matching using a predetermined list of keywords.

3. A computer-implemented method according to claim 2, wherein each of the predetermined list of keywords is extracted from at least one of domain knowledge and available standard ontologies.

4. A computer-implemented method according to claim 1, wherein acquiring the first finding from the radiology report is based on a trained model,
wherein the model is trained for identifying one or more findings from radiology reports, using at least one of machine learning processes and deep learning processes, and
wherein the model is trained based on a plurality of human annotated radiology reports, each of the plurality of human annotated radiology reports comprising one or more findings.

5. A computer-implemented method according to claim 4, wherein at least one of the machine learning processes and deep learning processes is based on one of: logistic regression, support vector machine, random forest, convolutional neural networks, and recurrent neural networks.

6. A computer-implemented method according to any one of the preceding claims, wherein the radiology report comprises a plurality of sections, each of the plurality of sections being associated with a finding or a type of information associated with the radiology report, and
wherein acquiring the first finding or the information associated with the radiology report comprises:
identifying a relevant section in the radiology report among the plurality of sections; and
using natural language processes to extract words and/or phrases from the identified section, wherein the words and/or phrases are relevant to the first finding or the information associated with the radiology report.

7. A computer-implemented method according to any one of the preceding claims, further comprising acquiring a clinical history of the subject, wherein determining whether the first finding is an incidental finding is further based on the acquired clinical history of the subject.

8. A computer-implemented method according to any one of the preceding claims, further comprising:
acquiring a second finding from a previously radiology report associated with the subject, wherein the second finding of the same pathological type as that associated with the first finding,
wherein determining whether the first finding is an incidental finding is further based on a comparison between the first finding and the second finding.

9. A computer-implemented method according to claim 8, further comprising:
acquiring, for each of the first finding and the second finding, one or more features, wherein each of the one or more features is indicative of a parameter of an observed element associated with the respective finding,
wherein the comparison between the first finding and the second finding comprises comparing the one or more features associated with the first finding with the one or more features associated with the second finding.

10. A computer-implemented method according to claim 9, wherein a parameter of an observed element is one of: an anatomical location of the observed element, a size of the observed element, a shape of the observed element, margin information of the observed element, a degree of opacity of the observed element, and a texture of the observed element.

11. A computer-implemented method according to any one of the preceding claims, wherein if it is determined that the first finding is an incidental finding, the method further comprises:
acquiring a clinical guideline, wherein the clinical guideline corresponds to the acquired information associated with the radiology report and comprises a decision support tool;
acquiring one or more features associated with the first finding, wherein each of the one or more features is indicative of a parameter of an observed element associated with the first finding; and
determining whether a follow-up action or a follow-up recommendation is required for the first finding, based on the acquired one or more features associated with the first finding and the decision support tool of the acquired clinical guideline.

12. A computer-implemented method according to claim 11, further comprising acquiring information associated with the subject, wherein determining whether a follow-up action or a follow-up recommendation is required for the first finding is further based on the acquired information associated with the subject.

13. A computer-implemented method according to any one of the preceding claims, wherein the modality of the imaging examination is one of: a computed tomography scan, a magnetic resonance imaging scan, an ultrasound scan, and an X-ray scan.

14. A computer program product comprising a computer readable medium, the computer readable medium having computer readable code embodied therein, the computer readable code being configured such that, on execution by suitable computer or processor, the computer or processor is caused to perform the method as claimed in any one of the preceding claims.

15. An apparatus for detecting an incidental finding in a radiology report associated with an imaging examination of a subject, the apparatus comprising a processor configured to:
acquire a first finding from the radiology report, the first finding being indicative of at least one of: an imaging observation, a disorder, and an abnormality identified from the imaging examination;
acquire information associated with the radiology report, wherein the acquired information associated with the radiology report comprises at least one of: a reason for performing the imaging examination, a modality of the imaging examination, and a body part of the subject examined in the imaging examination; and
determine whether the first finding is an incidental finding based on the acquired information associated with the radiology report, wherein the incidental finding is an unanticipated finding not related to a diagnostic inquiry associated with the imaging examination.
